(19) Europäisches Patentamt
European Patent Office
Office européen des brevets

(11) EP 1 438 041 B1

(12) **EUROPEAN PATENT SPECIFICATION**

(45) Date of publication and mention of the grant of the patent:
**28.11.2007 Bulletin 2007/48**

(21) Application number: **02770104.4**

(22) Date of filing: **24.10.2002**

(51) Int Cl.:
***A61K 31/4015*** *(2006.01)* ***A61P 15/16*** *(2006.01)*
***C07D 207/452*** *(2006.01)*

(86) International application number:
**PCT/GB2002/004813**

(87) International publication number:
**WO 2003/035059 (01.05.2003 Gazette 2003/18)**

(54) **SPERMICIDES**

SPERMIZIDE

SPERMICIDES

(84) Designated Contracting States:
**AT BE BG CH CY CZ DE DK EE ES FI FR GB GR IE IT LI LU MC NL PT SE SK TR**

(30) Priority: **25.10.2001 GB 0125659**

(43) Date of publication of application:
**21.07.2004 Bulletin 2004/30**

(73) Proprietor: **LRC PRODUCTS LIMITED**
**London EC4A 6BW (GB)**

(72) Inventors:
- **SOLANKI, Suren**
  **Cambridge**
  **Cambridgeshire CB4 0GZ (GB)**
- **POTTER, William, Duncan**
  **Cambridge CB2 5FD (GB)**
- **ANDERSON, Lorraine**
  **Edinburgh EH4 2SP (GB)**

(74) Representative: **Crooks, Elizabeth Caroline et al**
**Kilburn & Strode**
**20 Red Lion Street**
**London WC1R 4PJ (GB)**

(56) References cited:
**JP-A- 9 129 240**

- **MAMMOTO AKIKO ET AL: "Involvement of a sperm protein sensitive to sulfhydryl-depleting reagents in mouse sperm-egg fusion." JOURNAL OF EXPERIMENTAL ZOOLOGY, vol. 278, no. 3, 1997, pages 178-188, XP001068230 ISSN: 0022-104X**
- **REYES A ET AL: "INTERFERENCE WITH EPIDIDYMAL PHYSIOLOGY AS POSSIBLE SITE OF MALE CONTRACEPTION" ARCHIVES OF ANDROLOGY, vol. 7, no. 2, 1981, pages 159-168, XP001076674 ISSN: 0148-5016 cited in the application**
- **REYES, ALEJANDRO ET AL.: "Inhibition of capacitation and of the fertilizing capacity of rabbit spermatozoa by blocking membrane sulfhydryl groups" EXCERPTA MED. INT. CONGR. SER. (1976), 370(RECENT ADV. HUM. REPROD., PROC. INT. CONGR., 1ST, 1974), 322-4, XP008002954**
- **DEANA R ET AL: "EFFECTS OF CALCIUM CHELATORS DIVALENT CATIONS AND SULFHYDRYL REAGENTS ON CALCIUM UPTAKE AND MOTILITY OF BOVINE SPERMATOZOA" CELL CALCIUM, vol. 9, no. 3, 1988, pages 121-128, XP008002951 ISSN: 0143-4160**
- **NIVSARKAR, MANISH ET AL.: "Involvement of endometrial membrane sulphydryl groups in blastocyst implantation: sulphydryl groups as a potential target for contraceptive research" CONTRACEPTION, vol. 64, no. 4, 2001, pages 255-259, XP001075056**
- **KAVANAGH J P ET AL: "LEVAMISOLE AND OTHER DI AMINE OXIDASE EC-1.4.3.6 INHIBITORS AS INHIBITORS OF SPERM MOTILITY" ARCHIVES OF ANDROLOGY, vol. 7, no. 1, 1981, pages 51-62, XP008002953 ISSN: 0148-5016**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- LAMIRANDE DE E ET AL: "PARADOXICAL EFFECT OF REAGENTS FOR SULHYDRYL AND DISULFIDE GROUPS ON HUMAN SPERMCAPACITATION AND SUPEROXIDE PRODUCTION" FREE RADICAL BIOLOGY AND MEDICINE, ELSEVIER SCIENCE, XX, vol. 7, no. 25, 1 November 1998 (1998-11-01), pages 803-817, XP001074632 ISSN: 0891-5849
- SAVA, M. ET AL: "Synthesis and characterization of some bismaleimides containing ether groups in the backbone" MACROMOLECULAR CHEMISTRY AND PHYSICS (2001), 202(12), 2601-2605 , XP002249153
- MILANO J C ET AL: "BISMALEIMIDES ET POLYBISMALEIDMIDES A PONT ETHER ET THIOETHER - SYNTHESE, REACTIVITE ET TENUE THERMIQUE" MACROMOLECULAR CHEMISTRY AND PHYSICS, WILEY VCH, WEINHEIM, DE, vol. 197, no. 11, 1 November 1996 (1996-11-01), pages 3837-3849, XP000634318 ISSN: 1022-1352
- TAKEDA, SHINJI ET AL: "Synthesis and properties of bismaleimide resins containing ether bonds" JOURNAL OF APPLIED POLYMER SCIENCE (1988), 35(5), 1341-50 , XP001148874

## Description

The present invention relates to the use of maleimide derivatives as specified in the claims as spermicidal agents. In particular it relates to the use of maleimide derivatives as contact spermicides. The invention further provides novel compounds and their use in medicine. The invention also relates to spermicidal formulations containing maleimide derivatives.

Spermicides are chemical products which inactivate or kill sperm. In use, they are generally applied topically to the vagina prior to sexual intercourse. Spermicides are considered to be a convenient form of contraception as they are widely available without prescription and they can be self-administered. In contrast to the contraceptive pill, spermicides do not need to be applied on a regular basis, but only prior to intercourse, their effect is temporary and they have no hormonal side effects. Spermicides may be used alone, but are often used in combination with barrier methods of contraception.

Currently available spermicides include nonoxynol-9, octoxynol-9, menfegol and benzalkonium chloride, which are all classified as detergent spermicides. It has been suggested that the detergent-type spermicides can cause lesions of the vaginal and cervical epithelia.

Maleimides are a well known class of chemical compounds. Maleimide derivatives are known to have a wide variety of utilities, and find application in the biological and medicinal fields, as well as in general industrial use, for example in the preparation of polymers and inks.

Thus for example, USP 3,129,225 (United States Vitamin and Pharmaceutical Corporation) describes maleimido betaines said to have antimicrobial and antiparasitic activity as well as pharmacological activity, in particular the reduction of serum cholesterol.

German ALS 1,140,192 (United States Rubber Company) describes a novel bismaleimide with a $CH_2$-O-$CH_2$ bridge, and its use in vulcanisation, cross-linking of polymers and as fungicide for tomatoes.

USP 4,876,358 (Texaco Inc.) describes novel bismaleimides having a (poly)oxyethylene bridge, for use in preparing polymers.

USP 5,552,396 and WO 97/19080 (Eli Lilly) describe classes of N,N-bridged bisindolylmaleimides which are said to be inhibitors of protein kinase C, and to be useful for the treatment of diabetes, ischemia, inflammation, cns disorders, cardiovascular disease, dermatological disease and cancer.

USP 5,380,764 (Goedecke AG) describes bis-indolemaleinimides,said to be protein kinase C inhibitors useful for treating diseases of heart or blood vessels, inflammatory processes, allergies, cancer and degenerative damage of cns, diseases of the immune system and viral diseases, including diseases caused by retroviruses HTLV -I, -II, -III.

WO 96/09406 (The Government of the United States of America, represented by the Secretary of the Department of Health and Human Services) describes the use of disulfides, maleimides and a variety of other electron acceptor compounds in inactivating HIV-1 and other retroviruses.

Igarashi et al., J. Industrial Microbiology, 6 (1990) pp223-225, report that various N-alkylmaleimides show antibacterial and antifungal activity.

Reyes et al, Archives of Andrology, 7, (1981), pp159-168, discloses the use of N-(4-carboxy-3-hydroxy-phenyl)-maleimide (CPhM) to reduce the fertilising capacity of sperm. Incubation with a 10mM CPhM solution for 10 minutes resulted in the fertilising capacity of sperm being decreased from 80% to 17%. 10 minutes exposure is outside what is considered to be a clinically relevant timescale for a spermicide, that is, between 20 and 40 seconds following the addition of a putative spermicide (Sander FV and Cramer: A practical method for testing the spermicidal action of chemical contraceptives, Hum. Fertil., 1941, 6, 134-153 and Kleinman RL in "IPPF Medical Handbook" 2nd edition, London, 1964, International Planned Parenthood Federation).

Mammoto et al., Journal of Experimental Zoology, 278 (1997), pp. 178-188 descries the effects of sulfhydryl (SH)-depleting reagents on mouse sperm-egg fusion. SH-depleting reagents studied were N-ethyl-maleimide, $Na_2S_4O_6$ and 5,5'-dithiobis(2-nitro-benzoic acid).

Reyes et al., Excerpta Med. Int. Congr. Ser. (1976), 370 (Recent Adv. Hum. Reprod., Proc. Int. Congr. 1st, 1974) pp. 322-324 describes inhibition of capacitation and of the fertilizing capacity of rabbit spermatozoa by blocking membrane sulfhydryl groups, using the reagent N-4-carboxy-3-hydroxyphenylmaleimide (CPhM).

There is therefore a continuing need for spermicidal compounds which act quickly and efficiently to inactivate or kill sperm.

We have now surprisingly found that the maleimide derivatives of the present invention reduce human sperm motility. The compounds are therefore useful as spermicides. Furthermore, results from cell cytotoxicity assays suggest that the compounds do not cause significant damage to the vaginal and cervical epithelium.

In a first aspect therefore the present invention provides the use of a maleimide compound of the general formula (III) for the preparation of a composition for use in a method of contraception

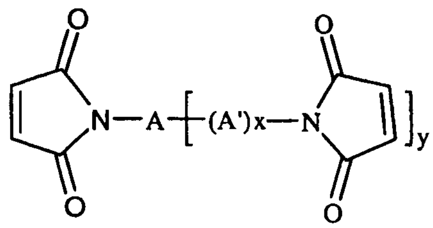

(III)

wherein

A is represented by $(R^1)_n$-$(R^2)_p$-$(R^3)_q$, wherein each of $R^1$, $R^2$ and $R^3$ is independently selected from:

an acyclic aliphatic group having one or from 3-20 carbon atoms;
a polyalkylene glycol group having an average molecular weight in the range 100 to 1000;
a cyclic aliphatic group having from 4-20 carbon atoms;
a monocyclic or polycyclic aromatic hydrocarbon group having from 6-18 ring carbon atoms;
a non-aromatic heterocyclic group having from 3 to 8 ring atoms, or
a monocyclic or polycyclic aromatic heterocyclic group having from 5 to 20 ring atoms;
and n, p and q each represent 0 or 1 such that the sum of n+p+q = 1-3;

A', which may be the same as or different from A, represents a group $(R^1)_n$-$(R^2)_p$-$(R^3)_q$, wherein $R^1$ , $R^2$ , $R^3$ , n, p and q are as defined above;
x is 0 or 1 and y is 1-6.

In the compounds of formula (III) above an acyclic aliphatic group or moiety may be branched or unbranched alkyl, which alkyl group may be optionally interrupted by one or more oxygen atoms; $C_{2-20}$ alkenyl; or $C_{2-20}$ alkynyl; any of which may be optionally substituted by one or more substituents selected from the group consisting of halogen, cyano, nitro, $-C(O)R^4$, $-CO_2R^4$, $-OR^4$, $-SR^4$-$SOR^4$, $-SO_2R^4$, - $NR^4R^5$, $-C(O)NR^4R^5$; wherein $R^4$ and $R^5$ independently represent hydrogen, $C_{1-8}$alkyl, aryl or aryl$C_{1-8}$alkyl.

For the purposes of the present invention, alkyl groups may have from 1 or 3 to 20 carbon atoms, preferably from 1 or 3 to 15 carbon atoms, more preferably from 1 or 3 to 10 carbon atoms. Alkenyl groups may have from 2 to 20 carbon atoms, preferably from 2 to 15 carbons atoms, more preferably from 2 to 10 carbon atoms. Alkynyl groups may have from 2 to 20 carbon atoms, preferably from 2 to 15 carbon atoms, more preferably from 2 to 10 atoms.

A polyalkyleneglycol group may be a polyethylene glycol (PEG) or a polypropylene glycol which may be represented by the formulae $-(OCH_2CH_2)_mOH$ or $-(OCH_2CH_2CH_2)_mOH$, wherein m is in the range 4 to 20. It will be appreciated that polyalkylene glycols are generally a mixture of chain lengths and are therefore conventionally described in terms of average molecular weight and average values of m. Thus for example PEG 200 has molecular weight in the range 190-210 and an average value of m=4 (The Merck Index, Eleventh edition).

Aryl is phenyl optionally substituted by one or more substituents selected from the group consisting of halogen, cyano, nitro, $-C(O)R^6$, $-CO_2R^6$, $-OR^6$, $-SR^6$-$SOR^6$, - $SO_2R^6$, $-NR^6R^7$, $-C(O)NR^6R^7$ wherein $R^6$ and $R^7$ independently represent hydrogen, or $C_{1-8}$alkyl.

A cyclic aliphatic group or moiety may be cycloalkyl or cycloalkenyl, either of which may be optionally substituted by one or more substituents selected from the group consisting of halogen, cyano, nitro, $-C(O)R^4$, $-CO_2R^4$, $-OR^4$, $-SR^4$-$SOR^4$, - $SO_2R^4$, $-NR^4R^5$, $-C(O)NR^4R^5$; wherein $R^4$ and $R^5$ are as defined above.

Cycloalkyl groups of the present invention may have from 3 to 20 carbon atoms, preferably from 3 to 10 carbon atoms, more preferably from 3 to 6 carbon atoms. Cycloalkenyl groups of the present invention may have from 3 to 20 carbon atoms, preferably from 3 to 10 carbon atoms, more preferably from 3 to 6 carbon atoms.

Aromatic hydrocarbon groups and moieties include for example optionally substituted unsaturated monocyclic, fused bicyclic or tricyclic rings of up to 18 carbon atoms, such as phenyl and naphthyl, and partially saturated bicyclic or tricyclic rings such as tetrahydro-naphthyl. Aromatic groups also include linked aromatic rings, such as a biphenyl group. The aromatic rings may also be linked by heteroatoms, e.g. O, S or $NR^4$. Examples of substituents which may be present on an aromatic group or moiety include one or more of halogen, alkyl, haloalkyl, cyano, nitro, $-C(O)R^4$, $-CO_2R^4$, $-OR^4$, $-SR^4$-$SOR^4$, $-SO_2R^4$, $-NR^4R^5$, or - $C(O)NR^4R^5$. Substituents on the aromatic ring are preferably in the ortho or para position with respect to the maleimido group.

An aromatic heterocyclic group or moiety may be unsaturated or partially saturated, for example an optionally substituted 5- or 6-membered heterocyclic aromatic ring which may contain from 1 to 4 heteroatoms selected from O, N and S. The heterocyclic ring may optionally be fused to one or more phenyl rings. Examples of heteroaryl groups thus

include furyl, thienyl, pyrrolyl, oxazolyl, oxazinyl, thiazolyl, imidazolyl, oxadiazolyl, thiadiazolyl, pyridyl, triazolyl, triazinyl, pyridazyl, pyrimidinyl, pyrazolyl, indolyl, indazolyl, benzofuranyl, benzothienyl, benzimidazolyl, benzoxazolyl, benzoxazinyl, quinoxalinyl, quinolinyl, quinazolinyl, cinnolinyl, benzothiazolyl, pyridopyrrolyl. Examples of substituents which may be present on a heterocyclic aromatic group include one or more of halogen, oxo, nitro, cyano, alkyl, haloalkyl, -C(O)$R^4$, -$CO_2R^4$, -$OR^4$, -$SR^4$-$SOR^4$, -$SO_2R^4$, - $NR^4R^5$, or -C(O)$NR^4R^5$, wherein $R^4$ and $R^5$ are as defined above. Partially saturated derivatives of the aforementioned heteroaryl groups include 3-pyrroline and 1,2-dihydroquinoline.

Non-aromatic heterocyclic groups include pyrrolidinyl, tetrahydrofuryl, tetrahydrothienyl and piperidinyl.

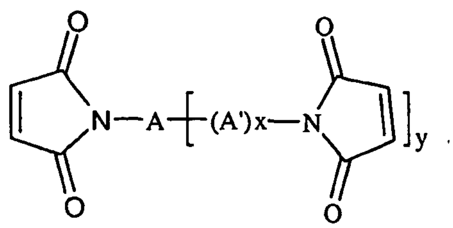

(III)

In compounds of formula (III) when x is 1, it is preferred that the total number of atoms in the moieties A and $A^1$ does not exceed 50.

A preferred class of compounds for use according to the invention is that of bismaleimide derivatives represented by formula (IV):

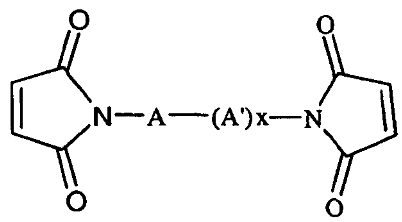

(IV)

wherein A, $A^1$ and x are as defined above.

In the compounds of formula (IV) x preferably represents 0 and A is as defined above.

In the group A, $R^1$, $R^2$, $R^3$, n, p and q are preferably selected such that A represents a $C_{1-8}$alkyl group, optionally interrupted by an oxygen atom;
a polyalkylene glycol group having an average molecular weight in the range 100 to 1000; or
a phenyl group; or
n and q are preferably each 1, p represents 0 or 1,$R^1$ and $R^3$ preferably each represent phenyl and, when p is 1, $R^2$ preferably represents a $C_{1-8}$alkyl group, optionally interrupted by an oxygen atom; or
a polyalkylene glycol group having an average molecular weight in the range 100 to 1000.

When A represents phenyl the maleimido moieties are preferably in the para position relative to each other. When A represents biphenyl the bonds between the maleimido and phenyl groups and the bond between the two phenyl groups are also preferably in the para positions.

Maleimide derivatives for use according to the present invention are commercially available or can be prepared according to methods well known in the art.

A maleimide derivative for use according to the present invention may be administered as the sole active ingredient or may be administered in combination with one or more other maleimide derivatives as defined herein and/or in combination with one or more other therapeutic agents. In this context 'in combination' means that the compounds may be administered simultaneously or sequentially, and there may be an interval of time between administration of the two or more compounds. Therapeutic agents which may be administered in combination with one or more maleimide derivatives include antibacterial, antifungal, and antiviral agents.

For use as spermicides the maleimide derivatives may be formulated for administration to the reproductive tract using conventional methods known in the art. Thus for example they may be formulated as creams, gels including thermoreversible gels lubricants, pessaries, foaming tablets, aerosol foams, sprays, douches or films. The compounds may be incorporated into sustained release formulations, e.g. by micro-encapsulation, formulation with a bio-adhesive material, such as poly-carbophil, or other conventional means. Gels, creams and pessaries may, according to circumstance, be administered via an appropriate applicator.

The formulations, in particular gels, creams and pessaries may be packaged with an appropriate applicator to facilitate administration.

Spermicidal preparations may also be incorporated into contraceptive devices. Thus for example they may be incorporated into vaginal, cervical or uterine devices such as vaginal sponges or rings, and diaphragms or used to lubricate and/or coat condoms. Spermicidal preparations used in this manner may be formulated for immediate and/or sustained release of the active compound(s) so as to provide controlled release over a period of time.

For use as a spermicide according to the present invention, a maleimide derivative may be administered at a dosage in the range 10-1000mg, e.g. 30 to 1000mg.

A second aspect of the invention provides a compound of formula (V)

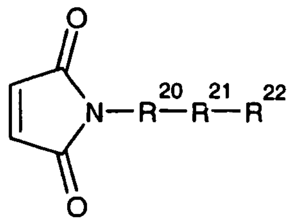

(V)

wherein $R^{20}$ is aryl, preferably phenyl;

$R^{21}$ is $-(OCH_2CH_2)_n-$ wherein n is 15;

$R^{22}$ is a group O-$R^{23}$ wherein $R^{23}$ is aryl; or

$R^{21}$ is $-(OCH_2CH_2)_n-$ wherein n is 3 to 15, preferably n is one of 4, 5, 6, 7, 8, 9, 10, 11, 12 and 13;

$R^{22}$ is a group O-$R^{23}$ wherein $R^{23}$ is $C_{1-10}$ alkyl, preferably $C_{1-4}$ alkyl, more preferably methyl, wherein the aryl or alkyl group is optionally substituted with

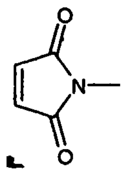

Preferred compounds for use in medicine are set out in the table below.

| Compound number | Structure |
|---|---|
| 13 | 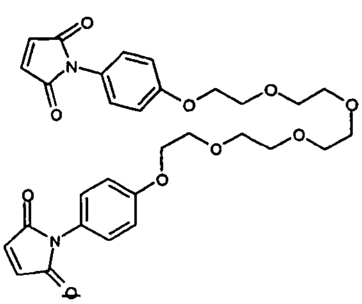 |

(continued)

| Compound number | Structure |
|---|---|
| 14 | 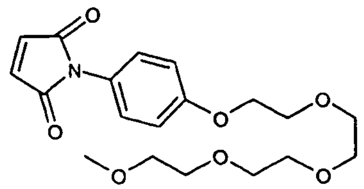 |
| 15 | 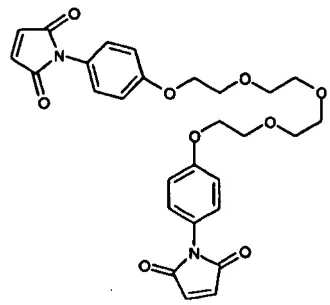 |
| 16 | 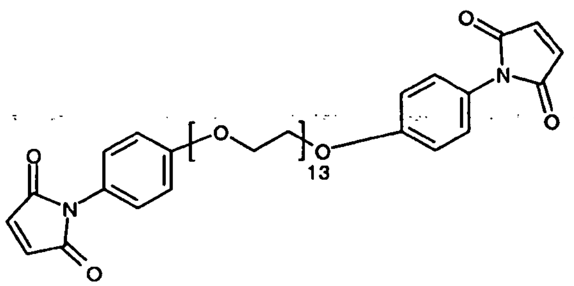 |

The second aspect of the invention also relates to salts, esters or amides of the compounds of formula V.

The compounds of the second aspect can be synthesised according to methods well known in the art. In particular, PEG of the appropriate chain length is initially converted to the aminoaryl derivative using conventional chemical synthesis. One example of such a chemical synthesis includes the coupling of PEG with p-fluoronitrobenzene in the presence of sodium hydride and DMF to produce a nitro-substituted aryl-PEG moiety. The nitro-subsituted aryl-PEG moiety is then reduced with a reducing agent such as palladium/carbon to form the required amine compound. The amine compound can then be converted into the maleimide compound of formula V by a number of methods known in the art. One example of such a method includes reacting the amine with maleic anhydride.

The third aspect of the invention provides a compound of formula V as defined above for use as a spermicide.

All preferred features of different aspects of the invention apply to each other *mutatis mutanalis*.

The invention will be further illustrated by the following examples:

## Examples

### Biological testing

### Methods

#### 1. Evaluation of Human Sperm Motility (spermicidal efficacy)

The number of motile sperm (%) is rapidly determined (within 30s) following exposure to varying concentrations of spermicide using manual microscopy. Using non-linear regression analysis motility data from these screens is used to generate concentration response curves and subsequently an $ED_{50}$ value using GraphPad Prism software (San Diego, CA, USA). The $ED_{50}$ value is the concentration of spermicide which produces 50% inhibition of sperm motility compared to control

**2. Determination of cell cytotoxicity (MTT Cell viability assay)**

The effect of compounds on vaginal cell viability is assessed using the MTT assay. MTT is a tetrazolium salt which is converted by viable cells to an insoluble tetrazoliym salt. This colour conversion is measured by spectrophotometry. Data is then subjected to non-linear regression analysis to generate concentration response curves and subsequently an $EC_{50}$ value curves using GraphPad Prism software (San Diego, CA, USA). The $EC_{50}$ value is the concentration of spermicide which produces 50% inhibition of cell viability compared to control.

**3. Determination of Therapeutic Index**

The therapeutic index is the ratio of cell cytoxicity $EC_{50}$ datum to the spermicidal efficacy $ED_{50}$ datum and is calculated using the data obtained in methods 1 and 2 above using the following formula:

$$\text{Therapeutic Index} = \frac{\text{Cell Cytotoxicity } [EC_{50}]}{\text{Spermicidal efficacy } [ED_{50}]}$$

**Test Compounds**

The following compounds of the invention were tested as set out in the above methods.

| Compound number | Structure |
|---|---|
| 1 | 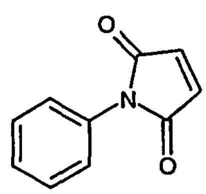 |
| 2 | 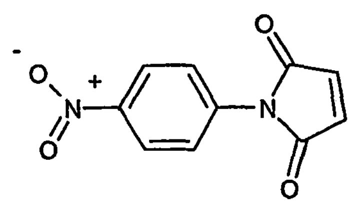 |
| 3 | 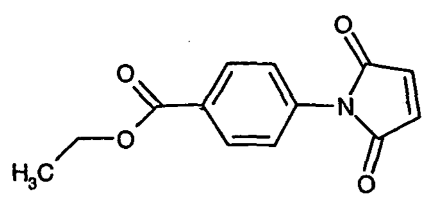 |
| 4 | 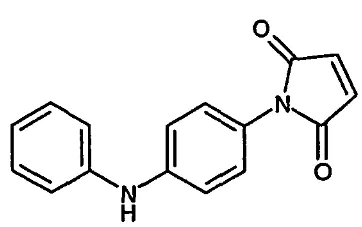 |

(continued)

| Compound number | Structure |
|---|---|
| 5 | 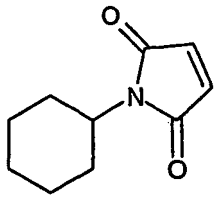 |
| 6 | 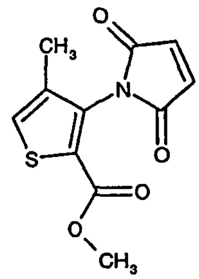 |
| 7 | 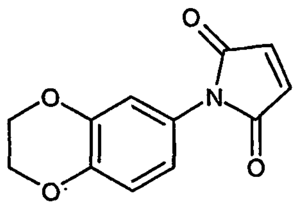 |
| 8 | 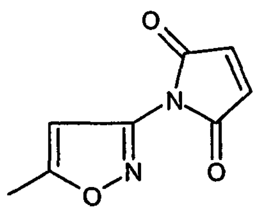 |
| 9 | 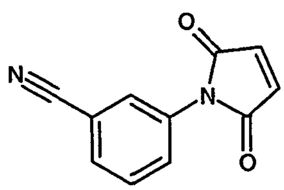 |
| 10 | 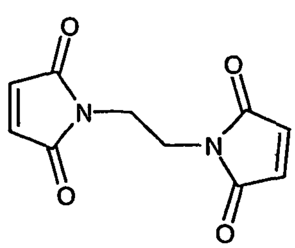 |

(continued)

| Compound number | Structure |
|---|---|
| 11 | 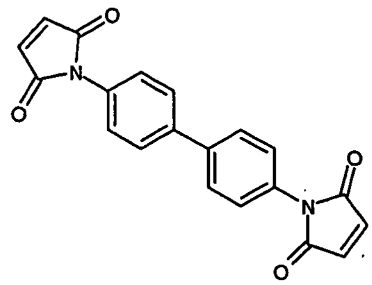 |
| 12 | 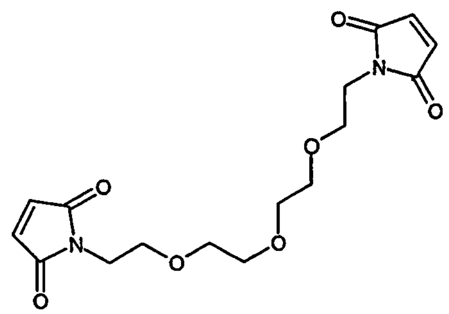 |
| 13 | 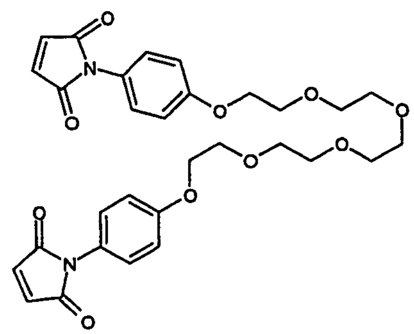 |
| 14 | 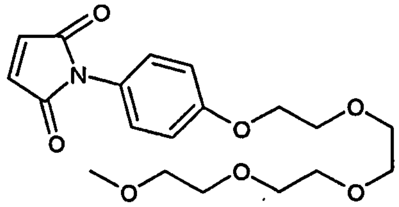 |
| 15 | 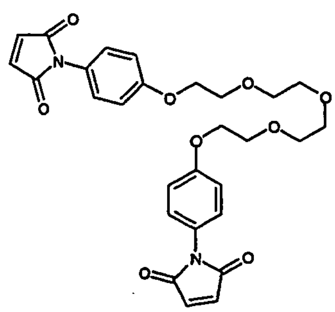 |

(continued)

| Compound number | Structure |
|---|---|
| 16 | 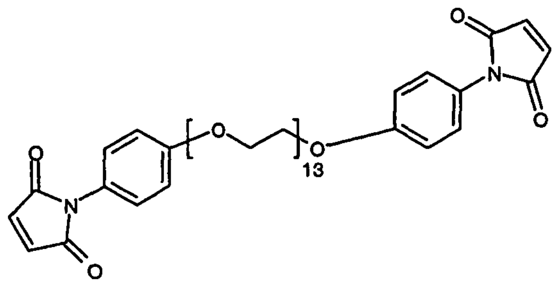 |

Compounds 1 and 5 were obtained from Sigma-Aldrich Corp, compounds 2 and 10 from Tokyo Chemical Industry UK, compounds 3, 6, 7, 8 and 9 from Maybridge plc, compound 4 from Wako Pure Chemicals Industries, Ltd, compound 11 from Sigma-Aldrich library of rare chemicals and compound 12 from Molecular Biosciences.

## Results

### 1. Effect of compounds on Human Sperm Motility

**Table 1**

| Compound | Inhibition Human Sperm Motility ($ED_{50}$- $\mu M$) |
|---|---|
| 1 | 32 (±5) n=3 |
| 2 | 26 (±7) n=3 |
| 3 | 77 (±10) n=3 |
| 4 | 31 (±3.5) n=8 |
| 5 | 294 (±24) n=6 |
| 6 | 26 (±1) n=3 |
| 7 | 43 (±3) n=3 |
| 8 | 79 (±16) n=3 |

(continued)

| Compound | Inhibition Human Sperm Motility ($ED_{50}$- μM) |
|---|---|
| 9 | 56 (±7) n=3 |
| 10 | 4 (±0.5) n=3 |
| 11 | 1.4 (±0.5) n=4 |
| 12 | 10.5 (±3.5) n=3 |
| 13 | 4.6 (±1.03) n=4 |
| 14 | 149 (±19) n=4 |
| 15 | 5 n=2 |
| 16 | 34.4 (±7.2) n=3 |

Table 1. Effect of Maleimides on Human Sperm Motility. Sperm motility was determined using manual microscopy. The concentration of each compound required to inhibit sperm motility ($ED_{50}$) by 50% was calculated from log concentration response curves using non-linear regression analysis. The number of experiments is quoted ± SEM.

**2. Effect of compounds on cell cytotoxicity**

**Table 2**

| Compound | Inhibition ME 180 Cell Viability ($EC_{50}$- μM) |
|---|---|
| 1 | 370 (±60) n=3 |
| 2 | 600 (±101) n=3 |
| 3 | 363 (±119.5) n=3 |
| | 268 |

(continued)

| Compound | Inhibition ME 180 Cell Viability ($EC_{50}$- μM) |
|---|---|
| 4 | (±126) n=7 |
| 5 | 114 (±41) n=3 |
| 6 | 88 (±28) n=4 |
| 7 | 410 (±85) n=4 |
| 8 | 858 (±142) n=3 |
| 9 | 143 (±25) n=3 |
| 10 | 516 (±106) n=3 |
| 11 | >30 (±) n=3 |
| 12 | 333 (±146) n=4 |
| 13 | 125 (±23) n=3 |
| 14 | 285 (±75) n=3 |
| 15 | 153 n=2 |
| 16 | 1890 (±1220) n=5 |

Table 2. Effect of Maleimides on ME180 Cell Viability. ME180 cell viability was determined using the MTT assay. The concentration of each compound required to inhibit ME180 cell viability ($EC_{50}$) by 50% was calculated from log concentration response curves using non-linear regression analysis. The number of experiments is quoted ± SEM.

**3. Therapeutic Index**

**Table 3**

| Compound | TI |
|---|---|
| 1 | 12 |
| 2 | 23 |
| 3 | 5 |
| 4 | - |
| 5 | 0.4 |
| 6 | 3 |
| 7 | 10 |
| 8 | 11 |
| 9 | 2.6 |
| 10 | 129 |
| 11 | >21 |
| 12 | 32 |
| 13 | 27 |
| 14 | 2 |
| 15 | 31 |
| 16 | 55 |

Conventional spermicides currently in use typically have therapeutic indices of 1 or less.

## Claims

1. The use of a maleimide compound of formula (III):

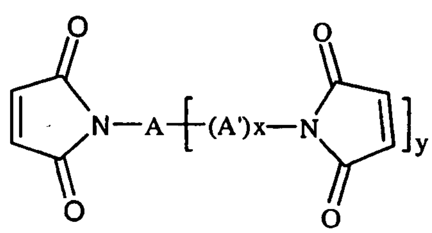

(III)

wherein A is represented by $(R^1)_n$-$(R^2)_p$-$(R^3)_q$, wherein each of $R^1$, $R^2$ and $R^3$ is independently selected from:

an acyclic aliphatic group having 1 or from 3-20 carbon atoms;
a polyalkylene glycol group having an average molecular weight in the range 100 to 1000;
a cyclic aliphatic group having from 4-20 carbon atoms;
a monocyclic or polycyclic aromatic hydrocarbon group having from 6-18 ring carbon atoms;
a non-aromatic heterocyclic group having from 3 to 8 ring atoms, or
a monocyclic or polycyclic aromatic heterocyclic group having from 5 to 20 ring atoms;
and n, p and q each represent 0 or 1 such that the sum of n+p+q = 1-3;
$A^1$, which may be the same as or different from A, represents a group $(R^1)_n$-$(R^2)_p$-$(R^3)_q$, wherein $R^1$ , $R^2$ , $R^3$ , n, p and q are as defined above;

x is 0 or 1 and y is 1-6;
for the preparation of a composition for use in a method of contraception.

2. Use according to claim 1 wherein the maleimide is a compound of formula (IV):

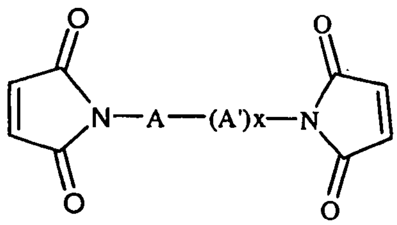

(IV)

wherein A, $A^1$ and x are as defined in claim 1.

3. A compound of formula V or a salt, an ester or an amide thereof.

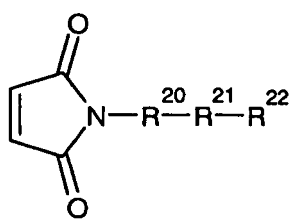

(V)

wherein $R^{20}$ is aryl;
$R^{21}$ is $-(OCH_2CH_2)_n-$ wherein n is 15;
$R^{22}$ is a group $O-R^{23}$ wherein $R^{23}$ is aryl; or
$R^{21}$ is $-(OCH_2CH_2)_n$ wherein n is 3 to 15;
$R^{22}$ is a group $O-R^{23}$ wherein $R^{23}$ is $C_{1-10}$ alkyl,
wherein the aryl or alkyl group is optionally substituted with

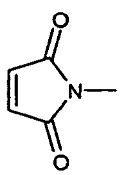

4. A compound as claimed in claim 3 selected from

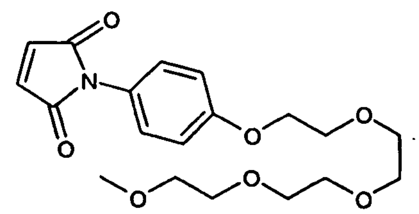

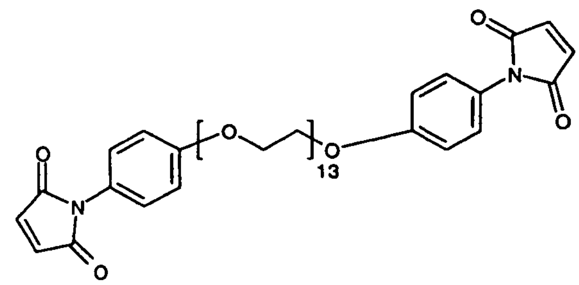

**5.** A compound of formula V or a salt, an ester or an amide thereof

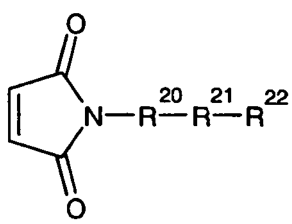

(V)

wherein $R^{20}$ is aryl;
$R^{21}$ is $-(OCH_2CH_2)_n-$ wherein n is 3 tol5;
$R^{22}$ is a group $O-R^{23}$ wherein $R^{23}$ is aryl or $C_{1-10}$ alkyl,
wherein the aryl or alkyl group is optionally substituted with

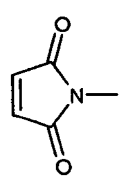

for use in medicine.

**6.** A compound selected from

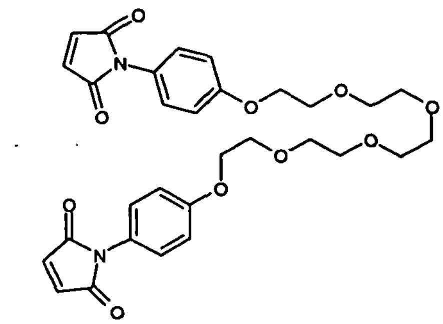

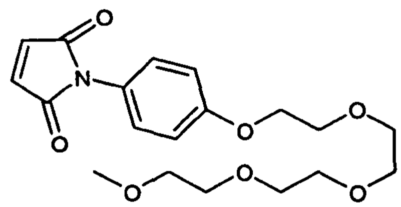

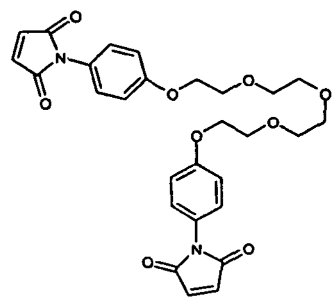

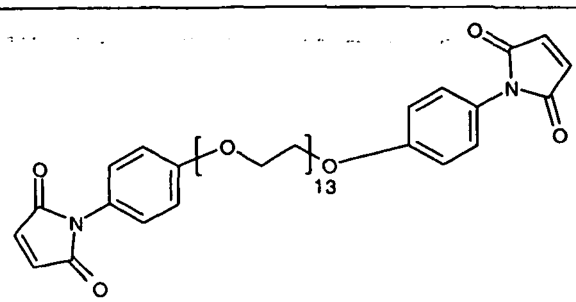

for use in medicine.

**7.** A compound as claimed in claim 3 or claim 4 for use as a spermicide.

## Patentansprüche

**1.** Die Verwendung einer Maleimid-Verbindung gemäß Formel (III) :

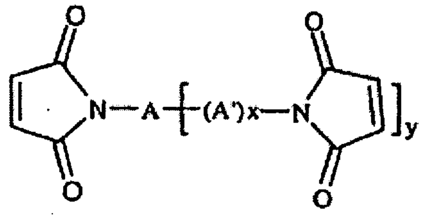

(III)

wobei A repräsentiert wird durch $(R^1)_n$-$(R^2)_p$-$(R^3)_q$, wobei $R^1$, $R^2$ und $R^3$ unabhängig voneinander ausgewählt sind aus:

einer acyclischen aliphatischen Gruppe mit 1, oder 3 bis 20 Kohlenstoffatomen;
einer Polyalkylenglykol-Gruppe mit einem durchschnittlichen Molekulargewicht in dem Bereich von 100 bis 1000;
einer cyclischen aliphatischen Gruppe mit 4 bis 20 Kohlenstoffatomen;
einer monocyclischen oder polycyclischen aromatischen Kohlenwasserstoff-Gruppe mit 6 bis 18 Ring-Kohlenstoffatomen;
einer nicht-aromatischen heterocyclischen Gruppe mit 3 bis 8 Ring-Atomen oder
einer monocyclischen oder polycyclischen aromatischen heterocyclischen Gurppe mit 5 bis 20 Ring-Atomen;
und n, p und q jeweils 0 oder 1 darstellen, so dass die Summe von n+p+q = 1-3 ist,
$A^1$, welches A entsprechen kann oder unterschiedlich sein kann, eine Gruppe $(R^1)_n$-$(R^2)_p$-$(R^3)_q$ repräsentiert, wobei $R^1$, $R^2$, $R^3$, n, p und q den oben Definierten entsprechen,
x 0 oder 1 und y 1 bis 6 ist;
für die Zubereitung einer Zusammensetzung für die Verwendung bei einem Kontrazeptionsverfahren.

**2.** Die Verwendung nach Anspruch 1, wobei die Maleimid-Verbindung die Formel (IV) aufweist:

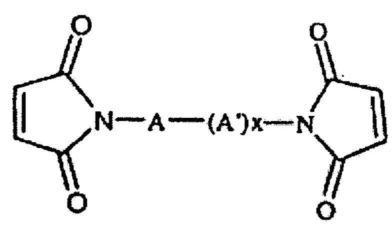

(IV)

wobei A, $A^1$ und x entsprechend Anspruch 1 definiert sind.

**3.** Eine Verbindung gemäß Formel V oder ein Salz, ein Ester oder ein Amid davon,

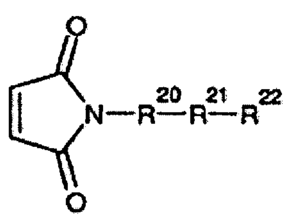

(V)

wobei $R^{20}$ ein Aryl ist;
$R^{21}$ -$(OCH_2CH_2)_n$- ist, wobei n 15 ist;

$R^{22}$ eine O-$R^{23}$-Gruppe ist, wobei $R^{23}$ ein Aryl ist; oder
$R^{21}$ -$(OCH_2CH_2)_n$ ist, wobei n 3 bis 15 ist;
$R^{22}$ eine O-$R^{23}$-Gruppe ist, wobei $R^{23}$ ein $C_{1-10}$-Alkyl ist,
wobei die Aryl- oder Alkyl-Gruppe optional substituiert ist mit

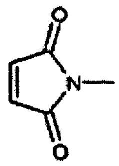

**4.** Eine Verbindung nach Anspruch 3, ausgewählt aus

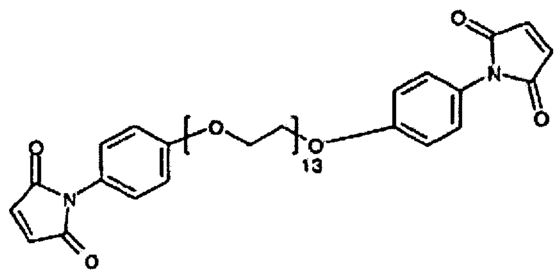

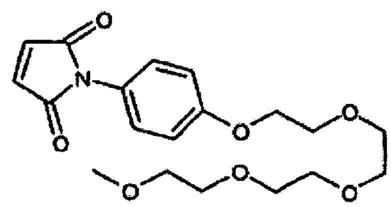

**5.** Eine Verbindung gemäß Formel V oder ein Salz, ein Ester oder ein Amid davon,

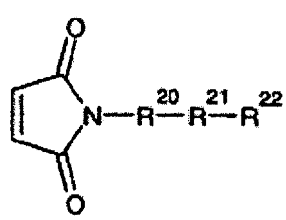

(V)

wobei $R^{20}$ ein Aryl ist,
$R^{21}$ -$(OCH_2CH_2)_n$- ist, wobei n 3 bis 15 ist,
$R^{22}$ eine O-$R^{23}$-Gruppe ist, wobei $R^{23}$ ein Aryl oder ein $C_{1-10}$-Alkyl ist,
wobei die Aryl- oder Alkyl-Gruppe optional substituiert ist mit

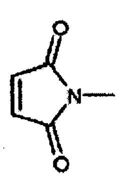

für die Verwendung in der Medizin.

**6.** Ein Verbindung ausgewählt aus

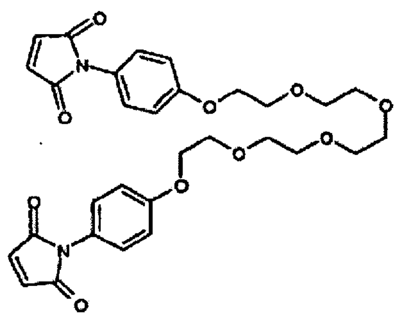

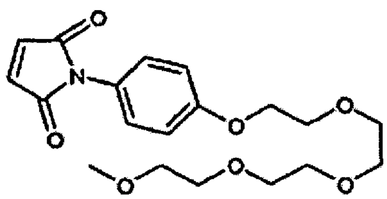

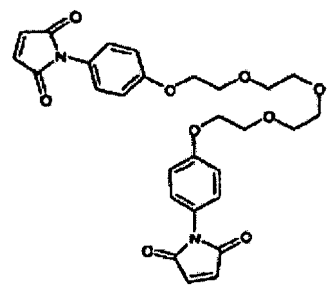

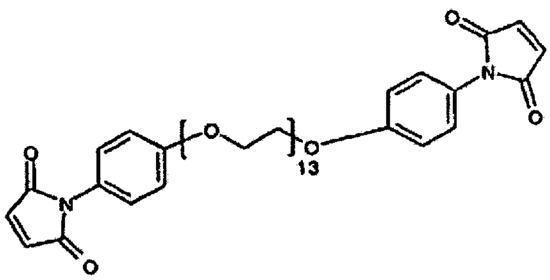

für die Verwendung in der Medizin.

**7.** Ein Verbindung nach Anspruch 3 oder 4 für die Verwendung als Spermizid.

## Revendications

**1.** Utilisation d'un composé de maléimide de formule (III) :

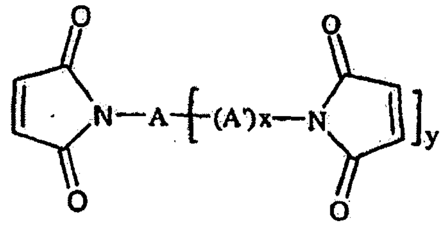

(III)

dans laquelle A représente $(R^1)_n$-$(R^2)_p$-$(R^3)_q$, où chaque radical $R^1$, $R^2$ et $R^3$ est choisi, indépendamment l'un de l'autre, parmi :

un groupement aliphatique acyclique ayant 1 ou 3 à 20 atomes de carbones ;
un groupement polyalkylène glycol ayant un poids moléculaire moyen compris dans la plage de 100 à 1000 ;
un groupement aliphatique cyclique ayant 4 à 20 atomes de carbone ;
un groupement hydrocarboné aromatique monocyclique ou polycyclique, ayant 6 à 18 atomes de carbone dans le cycle ;
un groupement non aromatique hétérocyclique ayant 3 à 8 éléments dans le cycle ; ou
un groupement aromatique hétérocyclique monocyclique ou polycyclique, ayant 5 à 20 atomes dans le cycle ;
et où n, p et q valent séparément 0 ou 1, de sorte que n + p + q = 1 à 3 ;
$A^1$, qui peut être identique ou différent de A, représente un groupement $(R^1)_n$-$(R^2)_p$-$(R^3)_q$, dans lequel $R^1$, $R^2$, $R^3$, n, p et q sont tels que définis ci-dessus ;
x est égal à 0 ou à 1 et y vaut 1 à 6 ;

pour la préparation d'une composition en vue d'une utilisation dans un procédé de contraception.

**2.** Utilisation selon la revendication 1, dans laquelle le maléimide est un composé de formule (IV) :

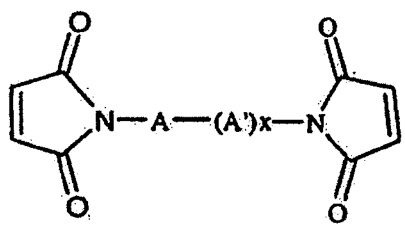

(IV)

dans laquelle A, $A^1$ et x sont tels que définis selon la revendication 1.

**3.** Composé de formule V suivante, ou un sel, un ester ou un amide de celui-ci :

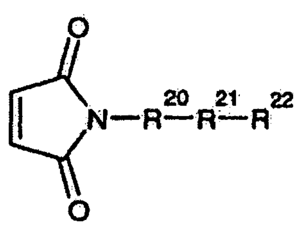

(V)

dans laquelle $R^{20}$ représente un aryle ;
$R^{21}$ représente $-(OCH_2CH_2)_n-$ où n est égal à 15 ;

$R^{22}$ représente un groupement O-$R^{23}$, dans lequel $R^{23}$ est un aryle ; ou
$R^{21}$ représente -$(OCH_2CH_2)_n$ où n vaut 3 à 15 ;
$R^{22}$ représente un groupement O-$R^{23}$, dans lequel $R^{23}$ représente un alkyle en $C_{1-10}$,
le groupement aryle ou alkyle étant éventuellement substitué par :

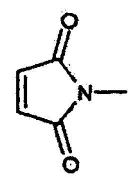

**4.** Composé selon la revendication 3, choisi parmi les composés suivantes :

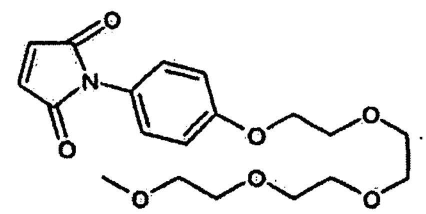

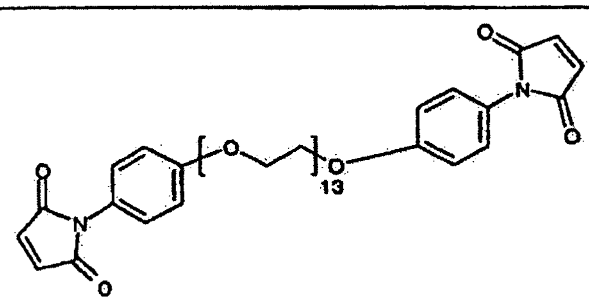

**5.** Composé de formule V suivante, ou un sel, un ester ou un amide de celui-ci :

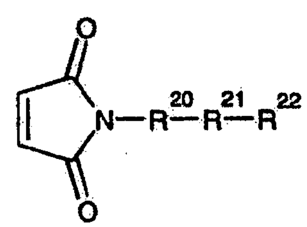

(V)

dans laquelle $R^{20}$ représente un aryle ;
$R^{21}$ représente -$(OCH_2CH_2)_n$ où n vaut 3 à 15 ;
$R^{22}$ représente un groupement O-$R^{23}$, dans lequel $R^{23}$ est un alkyle en $C_{1-10}$,
le groupement aryle ou alkyle étant éventuellement substitué par :

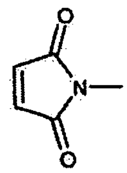

en vue d'une utilisation en médecine.

**6.** Composé choisi parmi la série de composés suivants :

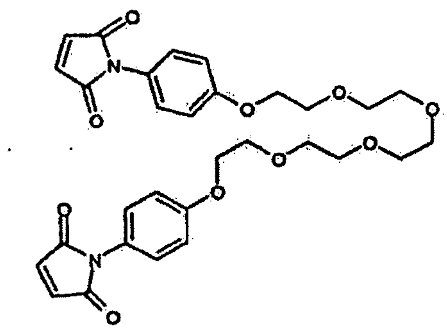

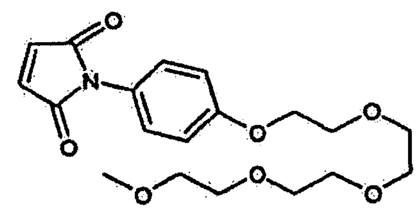

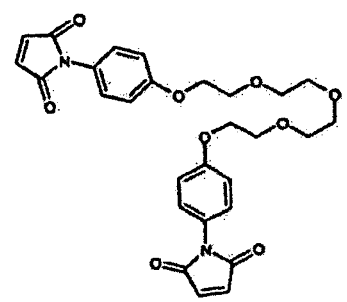

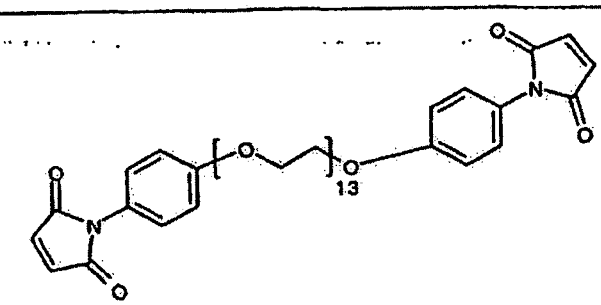

en vue d'une utilisation en médecine.

**7.** Composé selon la revendication 3 ou 4, pour une utilisation comme spermicide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3129225 A
- DE 1140192
- US 4876358 A
- US 5552396 A
- WO 9719080 A
- US 5380764 A
- WO 9609406 A


### Non-patent literature cited in the description


- **IGARASHI et al.** *J. Industrial Microbiology,* 1990, vol. 6, 223-225
- **REYES et al.** *Archives of Andrology,* 1981, vol. 7, 159-168
- **SANDER FV ; CRAMER.** A practical method for testing the spermicidal action of chemical contraceptives. *Hum. Fertil,* 1941, vol. 6, 134-153
- **KLEINMAN RL.** IPPF Medical Handbook. 1964
- **MAMMOTO et al.** *Journal of Experimental Zoology,* 1997, vol. 278, 178-188
- **REYES et al.** Excerpta Med. Int. Congr. Ser. *Recent Adv. Hum. Reprod., Proc. Int. Congr. 1st, 1974,* 1976, vol. 370, 322-324